# EUROPEAN PATENT APPLICATION

(11) **EP 2 987 451 A1**
(43) Date of publication of application: **24.02.2016**
(21) Application number: 14181316.2
(22) Date of filing: 18.08.2014
(51) Int. Cl.: A61B 5/00, G06F 19/00, A61B 5/16

(54) **Method and tools for predicting a pain response in a subject**

(71) Applicant: Tools 4 Patient sa, 6041 Gosselies (BE)
(72) Inventor: Pereira, Alvaro, 6220 Fleurus (BE); Gossuin, Chantal, 1640 Rhode-Saint-Genèse (BE); Demolle, Dominique, 5380 Cortil Wodon (BE); Gossen, Denis, 1950 Kraainem (BE); Helleputte, Thibault, 1300 Limal (BE)
(74) Representative: Brantsandpatents bvba

(57) **Abstract**

The current invention concerns a method; said method comprises collecting data via
- querying a subject on personality and/or health traits; and/or
- performing one or more social learning and/or (bio)physical tests by or on said subject;

characterized in that said data is used in a set of mathematical models thereby attributing one or more Scoring Factors to said subject, whereby said Scoring Factor is a measure of propensity of said subject to raise a response to a pain stimulus or a treatment strategy; and/or a measure of the intensity of said response of said subject.

## Description

### TECHNICAL FIELD

The invention pertains to the technical field of pain management and treatment. In particular, it relates to a methodology and tool for predicting a pain response in a subject.

### BACKGROUND

Pain is a unique brain response to a complex interplay between physiological phenomena and emotional and cognitive responses and is, thus, subject specific.

Physiological effects depend on a plurality of factors linked to the origin of pain such as metabolic (e.g. diabetes), toxic (e.g. chemotherapy or antiviral treatments), traumatic, surgical, neoplastic or infectious (e.g. zona) and psychological factors.

The physiological components of pain include mainly three dimensions: nociceptive pain, neuropathic pain, and inflammatory pain. The latter are well known in the art.

Pain may be classified according to origin of injury where it is at the peripheral and/or at the central nervous system.

Today, pain management, in particular in cancer strategies and approaches are either based on the clinician "best-guess" approaches based on its clinical practice and experience as well as on general guidance/recommendations established by key-opinion leaders and adopted by Health-Policy providers. These recommendations result primarily from a balance between pain relief observations and expectations at the level of a population of patients. Therefore, most often the caregivers stick to these population-based treatment schedules and/or recommendations.

For cancer pain for instance, a typical pain treatment is based on the use of specified analgesics, prescribed according to a pre-established schedule of drugs administration. Cancer pain management starts with the administration to the patient of mild analgesia (analgesics, pain-killers) and ends with strong morphine-like drugs. Adjuvants analgesics may also be used.

For focal neuropathic pain, a typical pain treatment is based on the use of lidocaïne, anti-depressants and antiepileptic compounds and secondly patches of capsaicin, tramadol and opiated.

For perioperative pain management, a typical pain treatment by anesthesiologist is based on the use options such as epidural or inthrathecal opioids, systemic opioid patient controlled analgesia or regional techniques including whenever possible, management therapy of NSAIDs, COXIBs, or acetaminophen at around the clock regimen or regional blockade with local anesthetics.

Although for each of these different pain domains, there seems to be a specific treatment or recommended protocol to follow, the results and rate of success are often variable and disappointing. The low success rate can be attributed to various factors.

The low success rates may result from a bad compliance of a patient to the recommended pain management protocols generally used in medical practice for relieving pain. To solve this problem, documents describe clinical decision support systems (CDSS) to correct deviations from pain therapy guidelines and increase the adherence to these guidelines (Bertsche et al., Pain, 2009, 147:20-28). Nevertheless the CDSS proposed in this document is not intended to describe the response of a subject to a pain stimulus or a pain management treatment or strategy; rather it is intended to increase the adherence to population-based pain management recommendations which are, although, not necessarily the treatment that is most suitable for relieving pain symptoms in a particular subject. The low success rates may thus also result from the observation that drugs and the protocols for using them (including the sequence of administration) are most often common for each patient for treating a pain-inducing disease or state. For these classical approaches the patient-specific nature of pain was not taken into account. Instead, these strategies were proposed from data and observations collected at the level of a population of patients. The low success rates may also result from the low level of knowledge regarding the general mechanisms explaining the onset and the evolution of pain. Pain is a complex result of the interplay of a plurality of factors including nociceptive, neuropathic and inflammatory mechanisms components. Not only are these mechanisms complex in nature but their impact on the expression of pain by a subject differs from subject to subject.

In addition, changes in the somatosensory system of subjects while submitted to pain stimuli and/or pain treatment, both peripherally and centrally may also give misleading or difficult-to-interpret data on the efficacy of a treatment or on the prediction of a patient-specific pain response. Thus low success rates may also result from the evolution in time of the response of a subject a pain stimulus or a pain treatment.

It has thus progressively been recognized that one of the main reasons of the disappointing results in the management pain come from the observation that the reaction to pain and to pain treatments is subject/patient-specific, time-dependent and disease-specific. Unfortunately, to date there are no known means to define either a clear picture of pain response at the level of the subject patient nor to predict these patient-specific reactions and their evolution in time.

Accordingly, there is also a need to design tailored pain relief therapies that specifically fit a subject's characteristics in terms of its propensity to favorably react to pain-relieving therapies.

Secondly, there is also a need for better pain management strategies whereby such strategies are based on appropriate multi-modular treatments including drug and non-drug therapies. Such therapies should be tailored to the response of a specific patient to pain and to pain treatment.

Better pain diagnostic and pain management strategies are needed as today they are impaired due to the weak level of reliable methodologies and tools for pain assessment that integrate the specific features of both subjects and pain mechanisms.

Few methodologies are proposed to unravel the mechanisms behind pain. US 2003 023 305 3 discloses a method whereby information from a patient regarding a pain or other sensation experienced by a patient is collected to determine one or more pain mechanisms derivable from the information. US 2003 023 3053 is hence oriented to deciphering the mechanisms associated to the symptoms and to solve the problem of identifying correctly the pain mechanisms underlying the expression of pain by a subject.

Documents exist describing the assessment of the level of pain and which is based on the activation of biophysical stimuli. As an example, WO 2006 039 416 describes an assay for the prediction of therapeutic efficacy for neuropathic pain. The method measures the level of pain activation by functional magnetic resonance imaging or other imaging methodologies.

Methods exist that propose adapting pain treatments to specific needs relying most often on a specific analysis of one given factor only, deemed to be a reliable measure of the evolution of pain in a subject. As an example, WO 2013 082 308 describes a method for personalized pain management by evaluating the presence or absence of a genetic determinant (single nucleotide polymorphism) in a subject. The method exclusively focuses on the presence/absence of the determinant and fails to take into account any other influential factor.

US 2013 021 797 6 describes an algorithm for evaluating the potential of an subject to develop pain after a surgical procedure, such as a hernia operation. The risk assessment is based on patient history and physical examination only.

WO 2014 059 278 describes a methodology for training a subject to accurately report pain in order to improve the accuracy of a clinical trial on pain management or for determination of pain treatment. WO 2014 059 278 does not describe a method for defining a pain profile of a patient in terms of its response to pain. None of the above-mentioned methodologies take into account the multi-factorial nature of pain, which largely depends on the specific patient.

As explained previously, attempts have been proposed to improve pain management of patients both by making recommendations and proposing population-based treatment protocols and by developing and applying decision tools for increasing the adherence to these recommendations. But the approaches fail to objectify the treatments because the reliable and multifactorial basis for such objectivized approach lacks.

As an attempt to solve the problem, US 5 908 383 describes a computer implemented method for generating a pain management treatment plan whereby said plan comprises inputting answers from a patient to questions in a computer program as well as demographic information, thereby computing a personalized treatment plan. The method is only focusing on patients already suffering from pain, and cannot be extended to healthy subjects or provide a predictive view on subjects, regardless whether they are currently suffering from pain or not. The method of US '383 does not allow the prediction of raising a response to a pain stimulus. The method as described in US 5 908 383 therefore rather provides a mere static image of the patient's pain, and not a dynamic one. Moreover, the method of US 5 908 338 is built around expert knowledge based data and decision trees, and is therefore not objective in its assessment.

There remains a need in the art for improved methodologies and algorithms for adequately predicting the development of a pain response in a subject, and, accordingly, (i) to better predict the response of a subject to a pain stimulus or a pain management treatment or strategy and (ii) to improve the pain treatment of a patient.

There is a need of developing methods and tools for establishing a subject's profile (signature, fingerprint) qualifying and quantifying a subject's propensity to raise a response to a pain stimulus or a favorable response to a pain management treatment.

### SUMMARY OF THE INVENTION

The present invention provides a method according to claim 1. The invention proposes to attribute one or more Scoring Factors to said subject whereby said Scoring Factor is a measure of the propensity of a subject to raise a response to a pain management treatment or to a pain stimulus. The method has the advantage that it combines (self) assessment of pain with biophysical or somatosensory tests for generating a patient specific pain profile, an approach that has to date not yet been used. The method does not pose a burden on the patient, and can hence be performed several times within a defined period, in order to adequately monitor the progression or to predict the evolution of pain profiles. This is ?? the contrary of currently used methods in the art.

The profile is meant to be used in conjunction with various models, in order to serve the purpose of several applications. As such, the method can be used for predicting the likelihood of a response of a subject to a pain stimulus according to claim 7 and for developing new or optimizing existing pain relief treatments in pain according to claim 8. Said method is preferably computer implemented. Hence the current invention also relates to a computer implemented product according to claim 6. Finally the current invention also relates a companion diagnostic tool according to claim 12, which can be used in addition to existing treatments, for clinical trial set-up, etc.

### DESCRIPTION OF FIGURES

Figure 1 shows a schematic overview of an embodiment of the methodology according to the current invention.
Figure 2 shows a screenshot of a computer interface according to an embodiment of the current invention, whereby scoring factors relating to a pain profile are computed based on input traits.

### DETAILED DESCRIPTION OF THE INVENTION

The present invention concerns a method for establishing one or more Scoring Factor(s) for a subject which can be used for various purposes within the field of pain management and treatment. The current invention aims to provide an all-embracing methodology, taking into account all relevant aspects which play a role in the perceiving of pain by a subject.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.
"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight" (weight percent), here and throughout the description unless otherwise defined, refers to the relative weight of the respective component based on the overall weight of the formulation.

In a first aspect, the current invention relates to a method based on collected data and an algorithm, whereby one or more Scoring Factors are computed, and whereby said Scoring Factor is a measure of propensity to raise a response to a pain stimulus or a treatment strategy; and/or a measure of the intensity of said response.

A response to pain or to pain stimuli depends highly in the subject and is a consequence of a plethora of factors, both relating to the physiological causes and effects of the pain stimuli as well as to factors inherently linked to the subject's traits (personality, health, environment, etc.). A method based on such multi-factorial approach employing objectivizing algorithms has to date not yet been described.

Moreover, none of the currently known methodologies are able to provide a predictive view on pain and the development of pain, regardless of the fact whether the subject is currently a patient with pain or a healthy subject.

For the purpose of the current invention, said Scoring Factor is a measure for a certain analyzed feature (in the current case the propensity for a subject to develop or experience pain upon given stimuli or a given pain management treatment). Said Scoring Factor may be a numerical factor, being an indication of the analyzed feature based on a specific scale, whereby the higher the numerical factor resides on the scale, the more likely it is that the analyzed feature is present. For example, in the context of the current invention, said Scoring Factor may provide a scale with regard to the propensity of an subject to be eligible for experiencing pain. In another embodiment, said Scoring Factor may be a classification or categorization of an analyzed feature in a certain subclass. For example, in the context of the current invention, said Scoring Factor may determine whether a subject is a responder or non-responder to a pain stimulus or a pain management treatment ('yes' or 'no'). In general, said Scoring Factor is a (predictive) value (e.g. a colour code, a definition, a term, a numerical factor...) of the subject's response to a pain stimulus or pain management treatment or natural disease evolution. In yet another embodiment, said Scoring Factor may be a measure of the pain intensity experienced by a patient, e.g. on a scale of 0 to 10, 0 being no pain, 10 being maximal pain.

In a preferred embodiment, one or more Scoring Factors will be attributed to said subject, whereby said Scoring Factor is a measure of propensity to raise a pain response and a measure of the intensity of the response. To that purpose, the data obtained is used in a mathematical model, the output of said model being the Scoring Factor. As such, a Scoring Factor is indicative for the pain profile of a subject or a component of a pain profile of a subject. Multiple Scoring Factors may be computed depending on their intended use afterwards.

In the context of the current invention, the term "Pain profile" is to be understood as the subject's specific "outcome of" or "response vis-à-vis" a pain stimulus or pain resulting from disease progression with or without the application of a pain management treatment or strategy, as expressed, perceived or measured by a subject for qualifying or quantifying either the improvement or the deterioration of his experienced pain upon a given stimulus or in the context of the administration of a given pain management treatment.

As meant here, "Pain profile", "pain fingerprint" or "pain signature" and "pain patterns" are synonyms.

In the context of the current invention, the terms 'predicting' and any derivatives thereof (predictive, prediction...) is to be understood as providing a probabilistic picture of an analysed feature (in the current context a pain profile), said picture is preferably computed by a model. Alternatively or in addition, predicting is to be understood as anticipating the evolution of said feature in time or during a predefined time period.

The term 'pain-related symptoms' is to be understood as subjective changes noticed or reported by the patient, e.g. pain provoked by a specific movement like walking or the temporal characteristics of pain attacks. Pain-related signs are objective findings that can be obtained by a clinician examining the patient, e.g. signs of inflammation like reddening or swelling.

In the context of the current invention, said pain stimulus or pain stimuli can be understood as any cause or origin of a pain sensation or observance in a subject, including e.g. a pain inducing disease.

In the context of the current invention, said "response" is to be understood as the outcome perceived by a subject as a reaction to stimuli or treatment given to said subject.

By preference, said response to a pain management therapy or strategy is favourable. A "favourable response" or "favourable clinical response" is to be defined as a pain relief treatment referring to a physiological and/or psychological response of a subject that is recognized by those skilled in the art as indicating a decreased expressed pain intensity compared to pain intensity that would be expressed by the subject with an alternate treatment or the absence of any treatment.

A response can for instance be analyzed on the basis of interviews with the subject, examination by a physician and/or pain testing. Such a favorable response may be a complete favorable response, i.e. showing an optimal improvement on all targeted aspects or symptoms, a partial response, i.e. showing some improvement on all targeted aspects or symptoms (regression) or a mixed response, i.e. where improvement is perceived on some but not all of the targeted aspects or symptoms.

More specifically, said method comprises a sequence of steps, whereby in a first step data is collected from a subject. In a second step, one or more combinations of the collected data are calculated, whereby said data comprises data from personality and/or health related queries and/or one or more pain-related (bio)-physical and/or one or more social behavior tests performed by or on said subject. In a more preferred embodiment, said collected data comprise any combination of two or three of the above-mentioned queries/tests. In a further, more preferred embodiment, said data includes data obtained by all the queries and tests mentioned above.

In the context of the current invention, 'trait or traits' is to be understood as all kinds of variables, whether or not directly linked to a subject, which can be input of the model according to the current invention, and which are used to come to one or more Scoring Factors. More in detail, said traits are identified by a skilled person based on current understanding of different aspects potentially related to pain, and commonly collected with existing questionnaires and/or tests. In a preferred embodiment, said trait variables can be numerical or categorical.

In the context of the current invention, 'personality traits' is to be understood as the characteristics of a subject which relate to the psychology of the subject, the physical characteristics of said subject and/or the personal background information of that subject. Said characteristics of the psychology may include, but are not limiting to emotional characteristics, behavioral characteristics, general beliefs of the subject and/or emotional traits. Said personality related queries are questions or series of questions which gauge the personality traits of a subject.

Said health traits may include all health related information of a subject, as well as of family of the subject. Said health traits may for instance include, but are not limiting to past and current diseases, received treatments, current and past medicinal use, potential health risks, genetic predisposition for disease development, etc. Said health related queries are queries or series of questions which gauge the health traits of a subject.

Within the context of the current invention, said social learning or social behavior might be understood as a process in which subjects observe the behaviour of others and its consequences, or specific situations and models to modify their own behaviour accordingly. Said social learning test includes providing a subject with behavioural, environmental and/or exemplary information or stimuli, thereby eliciting (or not) a response in said subject, based on the information received.

In various embodiments of the invention, the subject is a mammal, such as a human, a cat, a dog, a rodent, a primate, cattle, horses, etc. By preference said subject is human. More by preference, said subject is patient, preferably a patient suffering from a pain inducing disease.

Said pain may be caused by a functional illness which may include chronic pain. An exemplary chronic pain condition is neuropathic pain, which may include post herpetic neuralgia, HIV neuropathy, diabetic neuropathy, Fabry's disease, peripheral neuropathy, trigeminal neuralgia, post incisional neuropathic pain, phantom limb pain, reflex sympathetic dystrophy, causalgia, anesthesia dolorosa, intercoastal neuralgia, post-traumatic localized pain, complex regional pain syndrome, or neuropathic pain caused by trauma, lead, or chemotherapy.

By "acute pain" is meant pain of short duration that resolves completely and follows direct stimuli such as trauma (e.g., resulting from acute injury or surgery), inflammation, or burns. Typically, acute pain ceases when the stimulus is removed or the injured tissue has healed.

By "acute painful stimulus" is meant a stimulus that evokes acute pain in a subject. By "chronic pain" is meant persistent pain that is not caused by an acute stimulus. Most commonly, chronic pain results from a pathological condition such as infection, arthritis, chronic injury (e.g., sprain), cancer, migraine, irritable bowel disease, visceral pain disorders (e.g., chronic pancreatitis), and neuropathic pain. Chronic pain may also be idiopathic, e.g., fibromyalgia. Such pain may persist long after the inciting event.

By "neuropathic pain" is meant pain caused by peripheral nerve or central nervous system damage (e.g., stroke or spinal cord trauma). Neuropathic pain may include, without limitation, a burning sensation, hyperpathia, dysaethesia, allodynia, or phantom pain. Exemplary types of neuropathic pain include infective (e.g., post herpetic neuralgia and HIV neuropathy), metabolic (e.g., diabetic neuropathy and Fabry's disease), toxic (e.g., from lead or chemotherapy), traumatic/stretch injury (e.g., post incisional, trauma, phantom limb pain, and reflex sympathetic dystrophy/complex regional pain syndrome/causalgia), and idiopathic (e.g., trigeminal neuralgia).

By "therapeutic intervention or pain management strategy or pain management treatment" is meant a regimen intended to have a preventive, ameliorative, curative, or stabilizing effect. Examples of therapeutic interventions include pharmaceutical compositions, physical stimuli (e.g., massage or acupuncture), electrical stimuli, thermal stimuli, electromagnetic radiation, counseling, or a surgical, medical, or dental procedure.

The therapeutic intervention or pain management strategy may include a compound, a physical stimulus, an electrical stimulus, a thermal stimulus, electromagnetic radiation, counseling, or a surgical, medical, or dental procedure, or a combination thereof. The therapeutic intervention may also be administered subtherapeutically. Examples of acute painful stimuli include a change in temperature, mechanical force, a pin prick, or administration of a compound. The level of activation is measured, for example, by a neuroimaging device, such as a functional magnetic resonance device, positron emission tomography (PET) device, a magnetoencephalography (MEG) device, an electroencephalography (EEG) device, a single photon emission computer tomography (SPECT) device, an infrared (IR) device, a diffuse optical tomography (DOT) device, a magnetic resonance spectroscopy (MRS) device, or a functional computerized tomography (CT) device. When a control is used, the same subject may both be assayed by the methods described herein and serve as the control. The level of activation may be measured in one or more regions of the CNS, e.g., the orbital gyrus (Gob), ventral tegmentum/periaqueductal gray VT/PAG, nucleus accumbens (NAc), sublenticular extended amygdala (SLEA), cingulate gyrus, primary somatosensory cortex (S24), secondary somatosensory cortex (S2), thalamus, insula, cerebellum, prefrontal cortex, amygdala, hypothalamus, parahippocampal gyrus, hippocampus, entorrhinal cortex, ventral pallidum, dorsal striatum, primary motor cortices (M24), secondary motor cortices (M2), supplementary motor cortex (SMA), frontal eye field (FEF), rostral ventralmedial medulla (RVM), cerebellum, lateral prefrontal cortex, middle frontal gyri (Brodmann areas 44, 45, 46, 47), superior frontal gyri (Brodmann areas 6, 8), or brainstem subnuclei. Alternatively, the level of activation is not measured with reference to specific regions of the CNS.

There are many stand-alone questionnaires and pain tests available on the market, but to date none of them have been properly combined in order to derive a pain profile. The questionnaires known today (e.g. Brief Pain Inventory, Mc Gill Pain Questionnaire, Memorial Pain assessment, etc.) are not predictive and each of these questionnaires taken separately is meant (by those who created them) to provide a stand-alone pain self-assessment. In addition, todays biophysical/somatosensory tests (e.g. mechanical pain threshold, vibration deficits, etc.) are based on quantitative sensory testing (QST) evaluating mainly nociceptive pain and the neuropathic component of pain.

A mere juxtaposition of these known tests is not feasible, as the latter will not generate an adequate profile and will pose a too high burden on the subject (because of lengthy testing for instance).

The methodology proposed by the current invention allows a very fast analysis of a subject, gaining information on a pain profile of a subject in a minimum of time. This allows that the test is performed multiple times, even within a very short time span (e.g. less than 24 hours, a week, etc.).

In the context of the current invention, said (bio)physical test is to be understood as any test, relating to the measurement or detection of a biophysical parameter. For instance, said (bio)physical test may include but is not limited to measuring or analyzing a biological compound of said subject; measuring or detecting a biological reaction of said subject; performing a neurological test on said subject; measuring or detecting a sensory reaction; performing a tactile test on said subject.

By preference, said (bio)physical test involves a neurological, somatosensory, tactile or analytical test, or any combination thereof.

In a preferred embodiment, said (bio)-physical test takes changes in the somatosensory system into consideration. Amongst such tests are QST and other tests that may include heart rate monitoring, blood pressure monitoring, monitoring respiration, measuring one or more components or metabolites of blood (e.g. blood chemistry) or other bodily fluid, measuring skin parameters such as blood flow, temperature, or conductance; or other physiological measures including measuring any brain or neurological activity, skin conductance resonance (SCR), electroencephalography (EEG), quantitative EEG (QEEG), magnetic resonance imaging (MRI), functional MRI (fMRI), computed tomography (CT), positron emission tomography (PET), electronystagmography (ENG), single photon emission computed tomography (SPECT), magnetoencephalography (MEG), superconducting quantum interference devices (SQUIDS), electromyography, morphometric scanning of skin surfaces, eye movement tracking, and/ or pupillary diameter change, pain tests such as for instance heat pain procedure.

By preference, said (bio)physical test comprises a pain test or pain scoring test. Said test might be based on physical sensations or impressions such as heat stimuli, tactile stimuli e.g. by the van Frey monofilaments, by brush stroke, by a needle, by a pinprick, by cold stimuli, by vibrations e.g. applied to the skin.

The method according to the current invention implies questioning a subject on health and/or personality related issues. For the latter, specific personality queries are build, said personality queries comprise questions selected from clusters of questions or combinations of questions from different clusters.

These clusters of questions may be divided into the following groups or aspects.

In an embodiment, said personality query comprises one or more questions selected from clusters of questions for characterizing a subject's personality traits or characteristics which are stable over time and attributable to a person itself and not to the effect of its environment. Said cluster of questions related to personality comprises one or more questions for measuring the expectation, optimism" locus of control, anxiety, rumination, helplessness of personality, all of which are well-known to the skilled person in the art.

In another embodiment, said query comprises one or more questions selected from clusters of questions for measuring or evaluating the impact of a subject's surrounding on its perception of health-related issues.

Said cluster of questions related to the impact of the surrounding comprises:
- one or more questions for measuring the impact of the caregiver's behaviour (agreeable, open, severe..) or intervention (oral, acts...),
- one or more questions for evaluating the level of anxiety, fear, discouragement, depression related to the environment of a clinical setting or a caregiver.

In another embodiment, said query comprises one or more questions selected from clusters of questions for evaluating the impact of a subject's psychological quality of life, on life satisfaction, resistance to stress and depression...

In another embodiment, said query comprises one or more questions selected from clusters of questions for evaluating the attitudinal and emotional response of a subject to external stimuli. Said cluster of questions comprises questions for measuring the level of control that the subject believes to have on his life, the level control of external factors or health symptoms on his life such as luck, fate, life events or powerful others (such as e.g. relatives, health professionals, colleagues at work etc.) and for measuring the level of control of powerful others such as relatives on his attitude to resist, fight or overcome aggressive external factors or health symptoms.

In yet another embodiment, said such cluster of questions may comprise one or more questions for evaluating to which extent the caregiver estimates that health symptoms influence a patient's general physical and psychological condition comprising his body function, activity, mobility, working ability, relations with other people, sleep, life satisfaction, mood..., and to which extent the influence of the health symptoms on his general condition evolve with time.

In another embodiment, said query comprises one or more questions selected from clusters of questions for evaluating the level (severity) of pain. Said cluster of questions comprises one or more questions for measuring:
- to which extent the subject estimates that said pain influences his general physical and psychological condition comprising his body function, activity, mobility, working ability, relations with other people, sleep, life satisfaction, mood..., and to which extent the influence of the pain on his general condition evolve with time;
- to which extent the caregiver estimates that said pain influences a patient's general physical and psychological condition comprising his body function, activity, mobility, working ability, relations with other people, sleep, life satisfaction, mood..., and to which extent the influence of said pain on his general condition evolve with time.

In another embodiment, said query comprises one or more questions selected from clusters of questions for characterizing the typology and localisation of pain. Said cluster of questions comprises one or more questions for defining:
- the painful areas,
- how the subject translates pain in terms and qualifications such as painful cold, burning, electric shocks, mechanical shocks, tingling, pins, needles, numbness, itching etc.
- the physical status of the painful area such as hypoesthesia to touch, hypoesthesia to prick, pain caused or increased by mechanical actions on the body such as brushing, pinching etc.

In a further embodiment, said query comprises one or more questions chosen from any of the clusters of questions as outlined above. The clusters as described above may come in the form of questionnaires known in the art (e.g. locus of control, etc.) or may comprise questionnaires that are specifically designed by the inventors of the current invention.

A Scoring Factor relating to the pain profile of a subject will preferably be computed by a mathematical function of the input data. Said model will be built such that based on the input data, the Scoring Factor effect may be computed for each tested subject. Preferably, said model will be predictive. In an embodiment, said mathematical model will use Bayesian principles. In another embodiment, any other suitable mathematical model, known by a skilled artisan, can be used in the current invention. In a most preferred embodiment, said model is computer implemented.

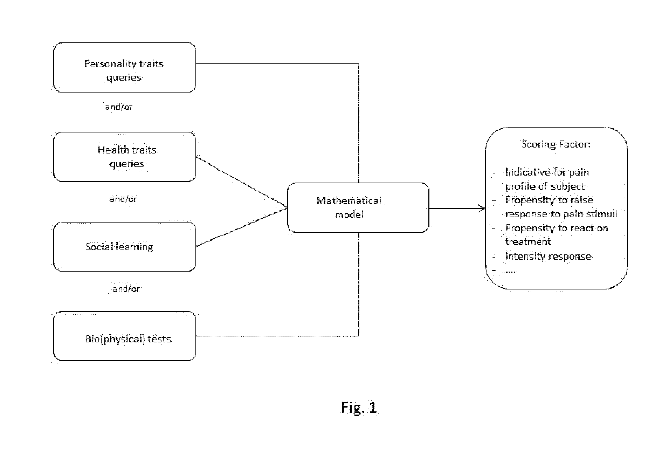

Figure 1 shows a schematic overview of an embodiment of the methodology according to the current invention.

Let P be a population defined by a n-row and p-columns matrix X of input data and a n-sized Y vector of observed placebo responses. Each of the n rows of X corresponds to a patient. Each of the p columns of X corresponds to a trait i.e. a personality trait. A signature S is defined as a subset of the p input traits. S is of size p' smaller or equal to p. S is used to define a new n-rows and p'-rows matrix called X' which together with Y defines P'.

A model estimation occurs on P'. The resulting model is called M. M is a function which maps a vector x of size p' to an output y. This output y is component of the pain profile evolution, in the current invention being the Scoring Factor.

### TRAITS

The p traits constituting the columns of matrix X described herein were identified by a skilled person based on current understanding of different aspects potentially related to pain, and commonly collected with existing questionnaires and/or tests. A person of the art will understand that the traits captured by such queries and/or tests might be captured as well by other but similar queries or tests. Thus queries and/or tests capturing the same traits but formulated differently than herein described may be employed in X as well instead of restricting the definition of X to the questionnaires and/or tests described above.

### TYPE OF PREDICTION

In one embodiment, entries of the Y vector are binary variables corresponding to respectively responders and non-responders to a pain stimulus or a pain management treatment/strategy.

In another embodiment, entries of the Y vector are ordinal variables with a finite number of modes corresponding to different response levels (for example non-responders, low responders, mild responders, strong responders) to a pain stimulus or a pain management treatment/strategy.

In another embodiment, entries of Y are continuous variables corresponding either to response likelihood or response intensity with respect to a pain stimulus or a pain management treatment/strategy.

In another embodiment entries of the y vector are categorical variables with a finite number of modes corresponding to different modes of response to a pain stimulus or a pain management treatment/strategy.

### MODEL

In one embodiment, the model M has the form of a linear model for regression or classification.

In another embodiment, the model M has the form of a k-Nearest Neighbour.

In yet another embodiment, the model M has the form of a decision tree.

In another embodiment, the model M is a set of models of the forms defined above built on various sub-samplings of the columns and or rows of P'. Alternatively, classification or regression can be achieved using other mathematical methods that are well known in the art.

In all cases, the sensitivity and specificity trade-off of the models can be tuned via a meta parameter according to the applicative context. The current invention covers all possible trade-offs.

As described herein, methods to predict a response to a pain stimulus or a pain management treatment/strategy or to identify subjects more likely to respond to a pain stimulus or a pain management treatment/strategy, is not meant to imply a 100% predictive ability, but is meant to indicate whether subjects with certain traits are more likely to experience a response to a pain stimulus or a pain management treatment/strategy than subjects who lack such characteristics. However, as will be apparent to one skilled in the art, some subjects identified as more likely to experience a response may nonetheless fail to demonstrate measurable response to a pain stimulus or a pain management treatment/strategy. Similarly, some subjects predicted as non-responders may nonetheless exhibit a response to a pain stimulus or a pain management treatment/strategy.

By preference, attribution of the Scoring Factor is computer implemented. The latter allows quick and accurate analysis of input data. In one embodiment, said attribution can be performed on a place remote from of the site of data collection. Said data can be obtained on one specific site and transferred to a second site (e.g. via electronic ways, systems stored in the cloud, etc.), where data analysis and Scoring Factor attribution occurs.

In the context of the current invention, the terms 'predicting' and any derivatives thereof (predictive, prediction...) is to be understood as providing a probabilistic picture of an analyzed feature, said picture is preferably computed by a model. Alternatively or in addition, predicting is to be understood as anticipating the evolution of said feature in time or during a predefined time period.

Hence, the current invention also relates to a computer implemented method for predicting a pain profile evolution of a subject. By preference, said computer implemented method comprises:
(a) inputting data obtained from personality and health-related queries, social learning and/or (bio)physical test performed by an subject;
(b) computing a measure of propensity to raise a response to a pain stimulus or a pain management treatment/strategy.

Additionally, the current invention also relates to a computer implemented product. Said computer product comprises at least one computer-readable storage medium having computer-readable program code portions stored therein, the computer-readable program code portions comprising instructions for computing a Scoring Factor for a subject, said Scoring Factor is linked to said subject and is a measure of propensity of said subject to raise a response to a pain stimulus or a treatment strategy; and/or a measure of the intensity of said response of said subject, whereby said Scoring Factor is based on subject-specific data, obtained from personality and/or health-related queries, and from pain-related (bio)-physical tests performed by on or said subject.

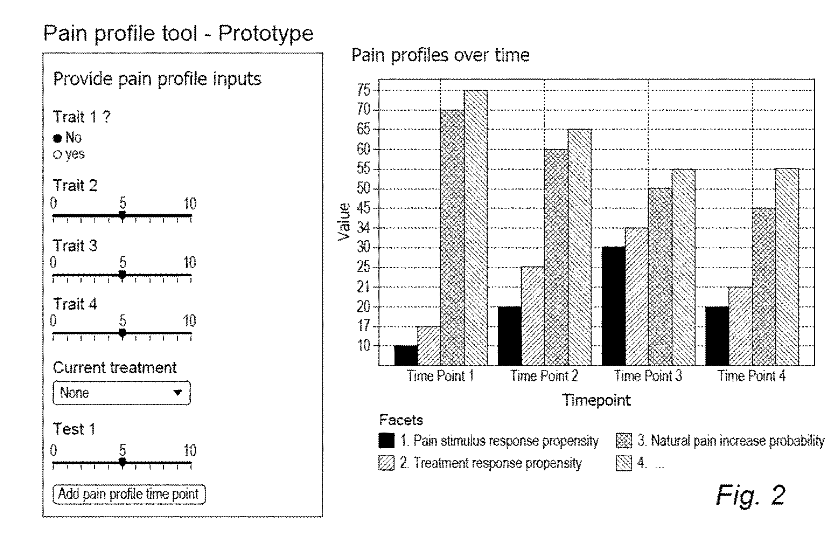

A schematic drawing of a possible embodiment of a computer implemented interface according to the current invention is shown in figure 2.

In a further embodiment, the input data from said subject, as well as the Scoring Factors thereof may be stored in a database; said database may be stored on an external server. Such database may serve for further analysis and for further fine-tuning of the algorithms and queries used for determining said Scoring Factor. In another embodiment, query or queries used are equally stored on an external server. The latter allows third parties to make use of the methodology and system, e.g. by remotely logging in to the system. In another more preferred embodiment, said database and queries are applicable for cloud computing and being stored and/or computed in the cloud.

In a preferred embodiment, the obtained Scoring Factor and optionally the imputed test and/or query results will be summarized in a report, said report may be a digital report sent to the person making use of the methodology.

### Time dependency

Not only is a pain profile subject-specific, it can also change during the course of time of a disease and/or treatment. The rating of pain intensity (or (self) assessment of pain) is also not constant with time for one specific patient. Pain scoring and accordingly Scoring Factors may vary with time depending of the evolution of the subject's personality traits and its health traits as well as with the pain management program provided to the subject patient. This raises additional concerns namely when clinical trials are conducted since the assessment of a new pain therapy will not necessarily be the same, for one subject patient, at the starting of the clinical trial or alongside its execution. Thus for performing clinical trials in any therapeutic indication for which an effective pain management is evaluated, there is a need to regularly reassess the current pain response of subjects included in the clinical trial (both in the experimental and the control arms of the study). Accordingly, such current pain profile should not represent an additional burden to both the patient and the caregiver. Because of the ease of the method described in the current invention, the method is suited for defining Scoring Factors for one subject over a (predefined) course of time, e.g. during a treatment or a clinical trial. Said multiple may be understood broadly, and may encompass several times a day (e.g. 2, 3, 4 times) or several times a week, a month, a year during a (predefined) period. Said time frame may for instance be the duration of a specific treatment or the duration of a clinical trial, or the duration of a disease (e.g. from point of diagnosis to cure), or during the entire lifespan of said subject. As such, multiple Scoring Factors for one subject can be generated during a time frame.

The generated Scoring Factors can be used as a measure for e.g. the propensity of said subject to develop a response to a pain stimulus and/or to a pain management therapy. Said response to a pain stimulus should be understood as a physiological and/or psychological reaction of a subject when subjected to a stimulus which is known to provoke pain or at least one pain component. Said pain component may be sensory, biological or psychological of nature.

### Improved Pain management strategies

The method of the current invention is specifically useful for predicting a pain profile of a subject or for predicting the propensity of a subject to respond to a pain management treatment. Hence, by implementing the current invention, pain treatment of a patient may be optimised, unnecessary treatments may be avoided and side-effects may be minimised. Therefore the current invention also relates to a method of identifying subjects for a therapeutic treatment based on their propensity to respond favourably to a pain management treatment, thereby predicting a Scoring Factor according to the method as described above.

A pain management therapy is to be understood as a detailed and personalized therapy, tailored to the needs of the subject for whom one or more Scoring Factors relating to a pain profile has been determined according to the method of the current invention. Said strategy may include one or more drug-related therapies (e.g. sort of drug, combination of various drugs, duration of the drug therapy, treatment schedules, dosage, mode of administration) as well as non-drug related therapies. Said non-drug related therapies may comprise physical therapy such as kinesitherapy, massage, transcutaneous electrical nerve stimulation (TENS) etc.; and/or complementary therapy such as relaxation, yoga, acupuncture, aromatherapy, etc. In a most preferred embodiment, said pain management therapy includes both drug and non-drug related therapies.

By preference, said response to a pain management therapy is favorable.

By performing the method according to the current invention, new pain relief treatments for pain inducing diseases or functional illnesses may be proposed as well as patient specific treatment plans. Moreover, existing pain relief treatments may be optimized.

As a corollary, the current invention will also provide the physician treating a patient with a proposal for a new or improved treatment or therapy. To date, mostly linear treatment plans are known and used whereby e.g. a standard treatment plan is used for every patient suffering from pain due e.g. by a specific illness. The WHO for instance recommends use of a "pain ladder" for cancer pain assessment, which is based on an analgesic treatment. In the field of focal neuropathic pain a typical pain treatment is based on the first line use of lidocaïne, anti-depressants and antiepileptic compounds, whereas in a second line patches of capsaicin, tramadol and opiates are prescribed. The rate of success of such linear treatment is often variable and disappointing as the latter do not take into account non-uniform reactions vis-à-vis pain and pain treatment. The current method aims to provide an alternative to these linear treatments, by e.g. suggesting other modes of administration and/or the use of co-therapies, etc.

The development and the evaluation of new analgesic drugs will also be facilitated by computing one or more Scoring Factors according to the current invention. Moreover, the design of clinical trials may be improved, by allowing the investigation of drug effects on specific mechanisms of pain, rather than attempting to measure overall reduction of pain.

### Improved clinical trials designs

In a further aspect, the method of the current invention may be equally used for selecting participants in a clinical trial. As used herein a "clinical trial" or "clinical study" is to be understood as encompassing all types of health-related studies in which obtaining data regarding safety and efficacy is a pre-requisite. As such, said clinical trial or study may refer to any research study, such as a biomedical or health-related research study, designed to obtain data regarding the safety or efficacy of a therapeutic treatment such as a drug, device, or treatment. Said clinical trial or study may equally relate to epidemiological or observational studies, market studies and surveys.

Assessment of Scoring Factors according to the current invention will provide tools to select and study populations of patients with specific pain profiles. Eventually, the current invention may radically change the design of pharmacological trials to study specific effects of drugs on pain mechanisms. Likewise, it will have an important impact on the design of investigations involving the significance of known mode of drug action as well as on alterations in the indication for use of particular analgesics determined in the label or package insert, allowed by regulatory authorities such as the Food and Drug Administration (FDA) or European Agency for the Evaluation of Medicinal Products (EMEA).

Generation of subject specific pain profiles taking into account all variables relevant for sensing or experiencing pain will improve the transfer of knowledge about the efficacy of compounds derived from animal models into clinical application as it provides a method to design drug trials based on a specific pain experience. Patients may be enrolled into trials only if they have a particular profile which can e.g. be linked to a particular target for a drug's action. As a consequence of the better selection methodology, the sample size necessary for the clinical trial may be considerably reduced by the method of the current invention. Moreover such optimized sample size selection will result in faster and more reliable decisions during the trials, which will have a positive impact on the ethical issues relating to clinical trials and the exposure of experimental compounds to patients.

### Companion diagnostic tools

In a further aspect, the current invention equally relates to a companion diagnostic tool. Said companion diagnostic tool is to be understood as a tool to predict whether a patient will respond to a certain pain management therapy. In an embodiment, said companion diagnostic tool according to the current invention is a companion diagnostic tool for predicting the response of a subject when applying a pain management strategy. Said tool preferably comprises instructions for computing one or more Scoring Factors for said subject, whereby said Scoring Factor is a measure of propensity to respond to a pain stimulus or a pain management treatment, based on subject-specific data obtained from personality traits and/or health traits and/or social learning tests and/or on or more (bio)physical tests performed by or on said subject.

The latter will help improve patient outcomes and decrease healthcare costs. For patients with a certain pain or pain-inducing disease, those that are identified as "not likely to respond to a certain pain management strategy" can quickly move on to other-perhaps more effective-therapies if they exist.

Furthermore, the companion diagnostic tool according to the current invention helps the healthcare system save costs by identifying the patient population that will most likely benefit from the therapy, and ruling out therapies that are not likely to be effective. This is especially important as some higher-priced therapeutics (e.g. for cancer) enter the market. An additional benefit can be realized by decreased costs related to managing side effects or hospitalizations due to unnecessary treatments.

In another aspect, said current invention relates to the use of the companion diagnostic tool as described above for patient specific treatment or for stratification of subjects in view of a clinical trial for a specific treatment.

As outlined above, the tool may be used for deciding on the optimal treatment of a patient. Secondly, said tool may also serve to classify/stratify subjects enrolled in a clinical trial or specific treatment. Prior to being enrolled in a clinical trial, the pain profile of an enrolled subject may first be evaluated, after which it may be decided in which group the subject may be categorized.

In another embodiment, said companion diagnostic tool will be useful as a tool for predicting whether or not, during a treatment or a trial, the outcome of the trial is biased or not due to the evolution during time of the pain profile of the enrolled subjects (pain profile shift or drift). The tool according to the current invention is fast and reliable, can be used multiple times throughout the course of the trial and is suitable for qualifying and/or quantifying a pain profile.

Finally, the current invention equally relates to a set of questions or queries, or a combination of the latter, for use in either a method as described above, or for a companion diagnostic tool as explained above.

Although the illustrative embodiments of the present invention have been described in greater detail, it will be understood that the invention is not limited to those embodiments. Various changes or modifications may be effected by one skilled in the art without departing from the scope or the spirit of the invention as defined in the claims.

## Claims

1. A method, said method comprises collecting data via
- querying a subject on personality and/or health traits; and/or
- performing one or more (bio)physical tests by or on said subject;
**characterized in that** said data is used in a set of mathematical models thereby attributing one or more Scoring Factors to said subject, whereby said Scoring Factor is a measure of propensity of said subject to raise a response to a pain stimulus or a treatment strategy; and/or a measure of the intensity of said response of said subject.

2. Method according to claim 1, **characterized in that** said Scoring Factor is computed by means of a mathematical model.

3. Method according to any one of the previous claims **characterized in that** said model is computer implemented.

4. Method according to any of the previous claims, **characterized in that** said personality query comprises questions selected from clusters of questions or combinations of questions from different clusters, said clusters of questions:
- relate to a subject's personality traits;
- measure or evaluate the impact of a subject's environment on health-related and/or psychological issues;
- evaluate the impact of a subject's quality of life;
- measure a subject's expectations, evaluate an subject's attitudinal and emotional response;
- evaluate to which extent the caregiver estimates that health symptoms influence a patient's general physical and psychological condition
- measure or evaluate the level of pain;
- characterize the typology and localization of pain; and
- evaluate the level of health symptoms of said subject.

5. Method according to any one of the previous claims, **characterized in that** said multiple Scoring Factors for one subject are generated during a time frame.

6. A computer implemented product, said computer program product comprising at least one computer-readable storage medium having computer-readable program code portions stored therein, the computer-readable program code portions comprising instructions for computing a Scoring Factor for a subject, said Scoring Factor is linked to said subject and is a measure of propensity of said subject to raise a response to a pain stimulus or a treatment strategy; and/or a measure of the intensity of said response of said subject, whereby said Scoring Factor is based on subject-specific data, obtained from personality and/or health-related queries, and/or from pain-related (bio)-physical tests performed by on or said subject.

7. A method for predicting the likelihood of a response of a subject to a pain stimulus, the method comprising the attribution of one or more Scoring Factors to a subject according to any one of the claims 1 to 5.

8. A method for predicting a response of a subject to a pain management therapy or for predicting the course and/or outcome of pain, the method comprising the attribution of one or more Scoring Factors to a subject according to any one of the claims 1 to 5.

9. A method for predicting a response of a subject to a pain management therapy according to claim 8, **characterized in that** said response is favorable.

10. Method for developing new or optimizing existing pain relief treatments in pain, the method comprising the attribution of one or more Scoring Factors to a subject according to any one of the claims 1 to 5.

11. Method according to claim 10, **characterized in that** said treatment includes drug and/or non-drug therapies.

12. A companion diagnostic tool, said tool comprises instructions for computing one or more Scoring Factors for a subject, said Scoring Factor is linked to said subject and is a measure of propensity of said subject to raise a response to a pain stimulus or a treatment strategy; and/or a measure of the intensity of said response in said subject, whereby said Scoring Factor is based on subject-specific data, said subject-specific data is obtained from personality and/or health-related queries and/or from one or more pain-related (bio)physical tests performed by or on said subject.

13. Use of a companion diagnostic tool according to claim 12 for proposing a patient specific pain relief treatment, for diagnosing and/or predicting a response, for forecasting the course and outcome of pain.

14. A set of questions or queries or combination of the latter for use in a method according to any one of the claims 1 to 5 or for use as a companion diagnostic tool according to claim 12.
